# EUROPÄISCHE PATENTANMELDUNG

(11) **EP 2 045 279 A1**
(43) Veröffentlichungstag der Anmeldung: **08.04.2009**
(21) Anmeldenummer: 07019563.1
(22) Anmeldetag: 05.10.2007
(51) Int. Cl.: C08G 18/08, C08G 18/12, C08G 18/28, C08G 18/32, C08G 18/40, C08G 18/44, C08G 18/48, C08G 18/72, C08J 9/30, C08K 3/00

(54) **Polyurethan-Schäume für die Wundbehandlung**

(71) Anmelder: Bayer MaterialScience AG, 51368 Leverkusen (DE); Bayer Innovation GmbH, 40225 Düsseldorf (DE)
(72) Erfinder: Schönberger, Jan, Dr., 42655 Solingen (DE); Mager, Michael, Dr., 51375 Leverkusen (DE); Dörr, Sebastian, Dr., 40597 Düsseldorf (DE); Rische, Thorsten, Dr., 59423 Unna (DE); Heckes, Michael, 47800 Krefeld (DE); Rudhardt, Daniel, Dr., 50823 Köln (DE); Gertzmann, Rolf, Dr., 51377 Leverkusen (DE); Dietze, Melita, 40699 Erkrath (DE); Fugmann, Burkhard, Dr., 40878 Ratingen (DE)
(74) Vertreter: Marx, Thiemo Patrick

(57) **Zusammenfassung**

Die Erfindung betrifft ein Verfahren zur Herstellung von Polyurethan-Schäumen für die Wundbehandlung, bei welchem eine Zusammensetzung, enthaltend eine Polyurethan-Dispersion und anorganische, kationische Koagulantien, aufgeschäumt und getrocknet wird.

## Beschreibung

Die Erfindung betrifft ein Verfahren zur Herstellung von Polyurethan-Schäumen für die Wundbehandlung, bei welchem eine Zusammensetzung, enthaltend eine Polyurethan-Dispersion und anorganische, kationische Koagulantien, aufgeschäumt und getrocknet wird.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in Regel Polyurethan-Schäume eingesetzt, welche durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind.

Eine Alternative zum vorstehend beschriebenen Verfahren, in welchem Diisocyanate bzw. NCO-funktionelle Polyurethan-Prepolymere eingesetzt werden, ist ein Verfahren basierend auf Polyurethan-Dispersionen (die im wesentlichen frei von Isocyanat-Gruppen sind), in welche man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen beschrieben in EP-A 0 235 949 und EP-A 0 246 723, wobei dem Schaum entweder ein selbsthaftendes Polymer zugesetzt oder der Schaum auf einen Film eines selbsthaftenden Polymers aufgebracht wird. Die in EP-A 0 235 949 und EP-A 0 246 723 aufgeführten Beispiele beschreiben darüber hinaus zwingend die Verwendung von Polyaziridinen als Vernetzer, was jedoch nach heutigem Stand des Wissens hinsichtlich deren Toxizität nicht mehr akzeptabel ist. Zur Vernetzung müssen überdies hohe Einbrenntemperaturen angewandt werden, angegeben sind 100°C bis 170°C. In US 4,655,210 ist die Verwendung der vorgenannten mechanischen Schäume für Wundauflagen beschrieben, die einen speziellen Aufbau aus Träger, Schaum und Hautkontakt-Schicht aufweisen. Die nach den EP-A 0 235 949 und EP-A 0 246 723 beschriebenen Verfahren hergestellten Schäume weisen darüber hinaus den großen Nachteil auf, dass die erhaltenen Schäume nur in geringem Maße offenzellig sind, wodurch die Absorption von physiologischer Salzlösung sowie auch die Wasserdampfdurchlässigkeit gering sind.

Zur Versorgung von Wunden mit komplexer Topologie oder zur Bedeckung besonders tiefer Wunden ist die Verwendung gebrauchsfertiger, industriell hergestellter flächiger Wundauflagen sehr schwierig, da eine optimale Bedeckung der Wundoberfläche in der Regel nicht gelingt, wodurch der Heilungsprozess verzögert wird. Um eine bessere Bedeckung tiefer Wunden zu erreichen, wurde vorgeschlagen, Granulate aus mikroporösen Polyurethanen anstelle kompakter Wundauflagen einzusetzen (EP-A-0 171 268). Jedoch wird auch hierdurch keine optimale Bedeckung der Wunde erreicht.

Der Auftrag einer (fließfähigen) Zusammensetzung, welche sich der Wundform optimal anpasst, würde die Nachteile flächiger Wundauflagen beseitigen. Die beiden vorstehend beschriebenen Verfahren, in welchen zur Herstellung der Polyurethan-Schäume entweder Diisocyanate bzw. NCO-funktionelle Polyurethan-Prepolymere oder Polyurethan-Dispersionen in Kombination mit Polyaziridinen eingesetzt werden, sind hierzu jedoch unbrauchbar: reaktive Zusammensetzungen, die freie Isocyanat-Gruppen enthalten, können nicht direkt auf die Haut aufgetragen werden, auch wenn dies verschiedentlich vorgeschlagen wurde (siehe beispielsweise WO 02/26848). Aber auch die Verwendung von Polyurethan-Dispersionen mit Polyaziridinen als Vernetzer ist aufgrund der nach heutigem Stand des Wissens toxikologisch bedenklichen Eigenschaften des Vernetzers ausgeschlossen.

Ein Verfahren zur raschen Verfestigung mechanisch geschäumter Polyurethan-Dispersion ist bislang nicht bekannt, auch wenn die Herstellung von Polyurethan-Filmen, d.h. nicht-porösen Materialien, durch z.B. Koagulation mit anorganischen Salzen ein weithin technisch gebräuchliches Verfahren ist.

Aufgabe der vorliegenden Erfindung war daher die Bereitstellung von Polyurethan-Schäumen für die Wundbehandlung, wobei die Herstellung unter Verwendung einer Zusammensetzung erfolgt, die frei von Isocyanat-Gruppen ist. Die Herstellung des Polyurethan-Schaums soll grundsätzlich auch bei Umgebungsbedingungen durchgeführt werden können, wobei die gebildeten Polyurethan-Schäume neben guten mechanischen Eigenschaften, eine hohe Absorption von physiologischer Salzlösung und eine hohe Wasserdampfdurchlässigkeit aufweisen sollen. Hierzu ist erforderlich, dass der Polyurethan-Schaum eine gewisse Offenzelligkeit aufweist. Die Zusammensetzung soll darüber hinaus zum direkten Auftrag auf die Haut geeignet sein, z.B. durch Sprühen oder Gießen, damit die Wunde optimal mit dem Polyurethan-Schaum bedeckt wird; eine schnelle Trocknung ist hierzu wesentlich.

Es wurde nun gefunden, dass man aus Zusammensetzungen, enthaltend Polyurethan-Dispersionen, welche frei von Isocyanat-Gruppen sind, und anorganischen, kationischen Koagulantien, bereits bei Umgebungsbedingungen Polyurethan-Schäume herstellen kann, die gute mechanische Eigenschaften, eine hohe Absorption von physiologischer Salzlösung und eine hohe Wasserdampfdurchlässigkeit aufweisen. Die Polyurethan-Schäume zeigen zumindest teilweise eine offenzellige Porenstruktur. Die fließfähigen Zusammensetzungen können überdies durch Sprühen oder Gießen direkt auf die Haut aufgebracht werden.

Gegenstand der vorliegenden Erfindung ist daher ein Verfahren zur Herstellung von geschäumten Artikeln, bevorzugt Wundauflagen, aus Polyurethan-Schäumen, bei dem eine Zusammensetzung enthaltend wässrige, anionisch hydrophilierte Polyurethan-Dispersionen (I) und anorganische, kationische Koagulanzien (II), aufgeschäumt und getrocknet wird.

Wundauflagen aus Polyurethan-Schäumen im Sinne der Erfindung sind poröse Materialien, bevorzugt mit zumindest teilweise vorhandener Offenzelligkeit, welche im wesentlichen aus Polyurethanen bestehen und Wunden im Sinne einer sterilen Abdeckung vor Keimen bzw. Umgebungseinflüssen schützen, eine schnelle und hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Die in den erfindungswesentlichen Zusammensetzungen enthaltenen wässrigen, anionisch hydrophilierten Polyurethan-Dispersionen (I) sind erhältlich, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol und
   A4) gegebenenfalls isocyanatreaktiven, anionischen oder potentiell anionischen und/oder gegebenenfalls nichtionischen Hydrophilierungsmitteln,
   hergestellt werden,
B) dessen freie NCO-Gruppen dann ganz oder teilweise
   B1) gegebenenfalls mit aminofunktionellen Verbindungen mit Molekulargewichten von 32 bis 400 g/mol und
   B2) mit isocyanatreaktiven, bevorzugt aminofunktionellen, anionischen oder potentiell anionischen Hydrophilierungsmitteln
   unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

Um eine anionische Hydrophilierung zu erreichen, müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder -SO₃⁻ oder - PO₃²⁻ als anionische bzw. deren ganz oder teilweise protonierte Säureformen als potentiell anionische Gruppen aufweisen.

Bevorzugte wässrige, anionische Polyurethan-Dispersionen (I) haben einen niedrigen Grad an hydrophilen anionischen Gruppen, bevorzugt von 0,1 bis 15 Milliäquivalenten pro 100 g Festharz.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 und 125 %, besonders bevorzugt zwischen 60 und 120 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C₁-C₈-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinentylglykolester, wobei Hexandiol(1,6) und Isomere, Neopentylglykol und entylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung 20 bis 80 Gew.-% und der Anteil an Polytetramethylenglykolpolyolen 80 bis 20 Gew.-% beträgt. Bevorzugt ist ein Anteil von 30 bis 75 Gew.-% an Polytetramethylenglykolpolyolen und ein Anteil von 25 bis 70 Gew.-% an Polycarbonatpolyolen. Besonders bevorzugt ist ein Anteil von 35 bis 70 Gew.-% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.-%, bevorzugt 60 Gew.-% und besonders bevorzugt mindestens 70 Gew.-% beträgt.

Die Verbindungen der Komponente A3) besitzen Molekulargewichte von 62 bis 400 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-εhydroxycapronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können in A3) auch monofunktionelle, isocyanatreaktive, hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO⁻M⁺, -SO₃⁻ M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol-% Ethylenoxid- und 0 bis 60 mol-% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketine von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin. Besonders bevorzugt werden Mischungen der vorgenannten Diamine der Komponente B1) eingesetzt, insbesondere Mischungen aus 1,2-Ethylendiamin und Isophorondiamin sowie Mischungen aus 1,4-Diaminobutan und Isophorondiamin.

Unter anionisch bzw. potentiell anionisch hydrophilierenden Verbindungen der Komponente B2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten Komponente A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen 1,05 bis 3,5, bevorzugt 1,2 bis 3,0, besonders bevorzugt 1,3 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (I) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (I) beträgt bevorzugt 40 bis 70, besonders bevorzugt 50 bis 65, ganz besonders bevorzugt 55 bis 65 und insbesondere 60 bis 65 Gew.-%.

Als anorganische, kationische Koagulantien (II) können in den Zusammensetzungen alle dem Fachmann an sich bekannten wasserlöslichen anorganischen Salze eingesetzt werden. Bevorzugte anorganische, kationische Koagulantien (II) sind Alkali- und Erdalkalisalze sowie Salze der 13. Gruppe des Periodensystems. Besonders bevorzugt sind die Salze der Erdalkalimetalle, ganz besonders bevorzugt sind die Salze von Magnesium und Calcium. Bevorzugt werden die Chloride, Sulfate und Phosphate der vorstehend beschriebenen Metall-Ionen eingesetzt. Im Einzelnen genannt seien Magnesiumchlorid, Calciumchlorid und Natriumchlorid.

Die anorganischen, kationischen Koagulantien können in fester Form oder als wässrige Lösungen eingesetzt werden. Bevorzugt ist die Verwendung von wässrigen Lösungen.

Neben den Polyurethan-Dispersionen (I) und den anorganischen Koagulantien (II) können auch Hilfs- und Zusatzstoffe (III) mitverwendet werden.

Beispiele für solche Hilfs- und Zusatzstoffe (III) sind Schaumhilfsmittel wie Schaumbildner und - stabilisatoren, Verdicker bzw. Thixotropiermittel, Antioxidantien, Lichtschutzmittel, Emulgatoren, Weichmacher, Pigmente, Füllstoffe und/oder Verlaufshilfsmittel.

Bevorzugt sind als Hilfs- und Zusatzstoffe (III) Schaumhilfsmittel wie Schaumbildner und - stabilisatoren enthalten. Geeignet sind beispielsweise handelsübliche Verbindungen wie Fettsäureamide, Sulfosuccinamide Kohlenwasserstoffsulfonate, -sulfate oder Fettsäuresalze, wobei der lipophile Rest bevorzugt 12 bis 24 Kohlenstoffatome enthält, sowie Alkylpolyglycoside, die nach dem Fachmann an sich bekannten Methoden durch Umsetzung von längerkettigen Monoalkoholen (4 bis 22 C-Atome im Alkylrest) mit Mono-, Di- oder Polysacchariden erhältlich sind (siehe z.B. Kirk-Othmer, Encyclopedia of Chemical Technology, John Wiley & Sons, Vol. 24, S. 29). Besonders geeignete Schaumhilfsmittel sind EO/PO-Blockcopolymere, die nach dem Fachmann an sich bekannten Methoden durch Addition von Ethylenoxid und Propylenoxid an OH- oder NH-funktionelle Startermoleküle erhältlich sind (siehe z.B. Kirk-Othmer, Encyclopedia of Chemical Technology, John Wiley & Sons, Vol. 24, S. 28). Zur Verbesserung der Schaumbildung, Schaumstabilität oder der Eigenschaften des resultierenden Polyurethan-Schaums, können neben den EO/PO-Blockcopolymeren noch weitere Additive in der Komponente (III) enthalten sein. Solche weiteren Additive können grundsätzlich alle an sich bekannten anionischen, nichtionischen und kationischen Tenside sein. Bevorzugt werden jedoch allein die EO/PO-Blockcopolymere als Komponente (III) eingesetzt.

Als Verdicker können handelsübliche Verdicker eingesetzt werden, wie Dextrin-, Stärke-, Polysaccharidderivate wie Gummi arabicum oder Cellulosederivate wie Celluloseether oder Hydroxyethylcellulose, organische vollsynthetische Verdicker, auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Grundsätzlich können, wenn auch nicht bevorzugt, die erfindungswesentlichen Zusammensetzungen auch Vernetzer wie unblockierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

Die erfindungswesentlichen Zusammensetzungen enthalten, bezogen auf Trockensubstanz, typischerweise 85 bis 99,5 Gewichtsteile der Dispersion (I), 0,5 bis 15 Gewichtsteile des anorganischen kationischen Koagulans (II), 0 bis 10 Gewichtsteile Schaumhilfsmittel, 0 bis 10 Gewichtsteile Vernetzer und 0 bis 15 Gewichtsteile Verdicker.

Bevorzugt enthalten die erfindungswesentlichen Zusammensetzungen, bezogen auf die Trockensubstanz, 85 bis 99,5 Gewichtsteile der Dispersion (I), 0,5 bis 15 Gewichtsteile des anorganischen kationischen Koagulans (II), 1 bis 7,5 Gewichtsteile Schaumhilfsmittel, 0 bis 5 Gewichtsteile Vernetzer und 1 bis 15 Gewichtsteile Verdicker, besonders bevorzugt, 70 bis 99,5 Gewichtsteile der Dispersion (I), 0,5 bis 15 Gewichtsteile des anorganischen kationischen Koagulans (II), 2,5 bis 7,5 Gewichtsteile Schaumhilfsmittel und 1 bis 15 Gewichtsteile Verdicker (bezogen auf die Trockensubstanz).

Neben den Komponenten (I), (II) und gegebenenfalls (III) können in den erfindungswesentlichen Zusammensetzungen auch andere wässrige Bindemittel eingesetzt werden. Solche wässrigen Bindemittel können z.B. aus Polyester-, Polyacrylat-, Polyepoxid- oder anderen Polyurethanpolymeren aufgebaut sein. Auch die Kombination mit strahlenhärtbaren Bindemitteln, wie sie z.B. in der EP-A-0 753 531 beschrieben sind, ist möglich. Ferner können auch andere anionische oder nichtionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Das Aufschäumen im erfindungsgemäßen Verfahren geschieht durch mechanisches Rühren der Zusammensetzung bei hohen Drehzahlen durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet, so dass eine Trocknung auf verletztem menschlichen oder tierischen Gewebe problemlos möglich ist. Für eine schnellere Trocknung und Fixierung der Schäume werden jedoch bevorzugt Temperaturen oberhalb von 30°C benutzt. Bei der Trocknung sollten jedoch Temperaturen von 200°C bevorzugt 150°C, besonders bevorzugt 130°C nicht überschritten werden, da es anderenfalls u.a. zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlern. Auch die Trocknung durch Führen des beschichteten Substrates über geheizte Oberflächen, z.B. Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren

Als Substrate geeignet sind insbesondere Papiere oder Folien, die ein einfaches Ablösen der Wundauflage vor ihrem Einsatz zur Abdeckung einer verletzten Stelle ermöglichen. Ebenso kann als Substrat menschliches oder tierisches Gewebe wie Haut dienen, so dass ein direktes Verschließen einer verletzten Stelle durch eine in-situ hergestellte Wundauflage möglich ist.

Ein weiterer Gegenstand der vorliegenden Erfindung sind die nach dem erfindungsgemäßen Verfahren erhältlichen Wundauflagen.

Die Polyurethan-Schäume haben vor ihrer Trocknung typischerweise Schaumdichten von 50 bis 800 g/Liter, bevorzugt 100 bis 500 g/Liter.

Die Polyurethan-Schäume besitzen nach ihrer Trocknung eine mikroporöse, zumindest teilweise offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³, und liegt besonders bevorzugt bei 0,01 bis 0,3 g/cm³.

Das Absorptionsvermögen gegenüber physiologischer Salzlösung beträgt bei den Polyurethan-Schäumen typischerweise 100 bis 1500 %, bevorzugt 300 bis 1500 %, (Masse der aufgenommen Flüssigkeit bezogen auf die Masse des trockenen Schaums; Bestimmung nach DIN EN 13726-1, Teil 3.2). Die Durchlässigkeit gegenüber Wasserdampf beträgt typischerweise 500 bis 8000 g/24 h * m², bevorzugt beträgt sie 500 bis 5000 g/24 h * m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,2 N/mm² und die maximale Dehnung ist größer als 200 %, bevorzugt ist die maximale Dehnung größer als 250 % (Bestimmung nach DIN 53504).

Die Polyurethan-Schäume haben nach dem Trocknen typischerweise eine Dicke von 0,1 mm bis 50 mm, bevorzugt 0,5 mm bis 20 mm

Die Polyurethan-Schäume können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Sofern es zweckdienlich ist, kann im erfindungsgemäßen Verfahren ein Schritt zur Sterilisation erfolgen. Ebenso ist es grundsätzlich möglich, die nach dem erfindungsgemäßen Verfahren erhältlichen Wundauflagen nach deren Herstellung zu Sterilisieren. Zur Sterilisation kommen die dem Fachmann an sich bekannten Verfahren zum Einsatz, bei denen eine Sterilisation durch thermische Behandlung geeignete Chemikalien wie Ethylenoxid oder Bestrahlung beispielsweise durch Gammabestrahlung erfolgt.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Bevorzugte Wirkstoffe der vorgenannten Art sind solche aus der Gruppe der Antiseptika, Wachstumsfaktoren, Proteaseinhibitoren und nichtsteroidalen Antiphlogistika/Opiate.

In einer bevorzugten Ausführungsform der vorliegenden Erfindung umfasst der Wirkstoff ein antiseptisches Biguanid und/oder dessen Salz, vorzugsweise das Hydrochlorid.

Als Biguanide werden Verbindungen bezeichnet, die sich vom Biguanid (C₂H₇N₅) ableiten, insbesondere dessen Polymere. Antiseptische Biguanide sind solche Verbindungen, die eine antimikrobielle Wirkung aufweisen, also als Bakteriostatika oder vorzugsweise als Bakterizide wirken. Die Verbindungen weisen vorzugsweise eine breite Wirkung gegen viele Bakterien auf und können durch eine Wirkung gegen E. coli von mindestens 0,5 µg/ml, vorzugsweise mindestens 12 oder mindestens 25 µg/ml gekennzeichnet werden (minimal mikrobizide Konzentration oder MMK, gemessen im Suspensionsversuch).

Ein bevorzugtes antiseptisches Biguanid gemäß dieser Erfindung ist Poly(imino[iminocarbonyl]iminopolymethylen), wobei die Verwendung von Poly(hexamethylen)biguanid (PHMB), das auch als Polyhexanid bezeichnet wird, als antiseptisches Biguanid besonders bevorzugt ist.

Der Begriff "antiseptische Biguanide" gemäß dieser Erfindung beinhaltet auch Metaboliten und/oder Prodrugs der antiseptischen Biguaniden. Antiseptische Biguanide können dabei als Racemate oder reine Isoformen vorliegen.

Bevorzugt enthalten die geschäumten Artikel aus Polyurethan-Schäumen beziehungsweise die Zusammensetzungen gemäß der vorliegenden Erfindung antiseptisches Biguanid und/oder dessen Salz, vorzugsweise das Hydrochlorid, in einer Konzentration von 0,01 bis 20 Gew-%, wobei die Konzentration von 0,1 bis 5 Gew-% besonders vorteilhaft ist. Das Biguanid kann eine beliebige Molekulargewichtsverteilung aufweisen.

Aufgrund der breiten Einsetzbarkeit des erfindungsgemäßen Verfahrens und der darüber zugänglichen Schäume und Wundauflagen ist es grundsätzlich möglich dieses in der industriellen Herstellung von Schäumen und Wundauflage einzusetzen. Ebenso ist es aber auch möglich dieses zur Herstellung z.B. von Sprühpflastern einzusetzen, wobei die Wundauflage durch direkten Auftrag der Zusammensetzung auf eine Wunde und gleichzeitiges Aufschäumen und anschließendes Trocknen gebildet wird.

Zur industriellen Herstellung von Schäumen und Wundauflagen wird die Polyurethan-Dispersion (I) mit Schaumhilfsmitteln der vorstehend genannten Art vermischt, danach mechanisch durch Eintrag eines Gases wie Luft aufgeschäumt und schließlich durch Zugabe des anorganischen, kationischen Koagulans (II) koaguliert, wobei ein weiterhin verarbeitbarer, koagulierter Schaum erhalten wird. Dieser Schaum wird auf eine Unterlage aufgetragen und getrocknet. Aufgrund höherer Produktivität erfolgt die Trocknung typischerweise bei erhöhten Temperaturen von 30 bis 200°C, bevorzugt 50 bis 150°C, besonders bevorzugt 60 bis 130°C. Die Trocknung erfolgt in der Regel unter Verwendung von an sich bekannten Heiz- und Trockenapparaten, z.B. (Umluft-) Trockenschränken. Der Auftrag sowie die Trocknung können jeweils diskontinuierlich oder kontinuierlich durchgeführt werden, bevorzugt ist jedoch ein gänzlich kontinuierliches Verfahren.

Bei der Verwendung der erfindungswesentlichen Zusammensetzung bei der Herstellung eines Sprühpflasters werden die Polyurethan-Dispersion (I) und das anorganische, kationische Koagulans (II), welche jeweils gegebenenfalls Schaumhilfsmittel enthalten können, getrennt voneinander bereitgestellt und unmittelbar vor oder beim Auftrag auf das abzudeckende Gewebe miteinander gemischt. Das Aufschäumen geschieht hier durch gleichzeitige Entspannung eines Treibgases, welches in mindestens einer der Komponenten (I) und (II) enthalten war. Zur Verfestigung wird der gebildete Schaum anschließend getrocknet, wobei Temperaturen von 20 bis 40°C bereits ausreichen. Unter Zuhilfenahme zusätzlicher Wärmequellen wie Fön oder IR-Rotlichtlampe ist allerdings auch eine forcierte Wärmetrocknung bis maximal 80°C möglich.

Die dabei eingesetzten Treibmittel sind an sich aus der Polyurethanchemie bekannt. So sind beispielsweise geeignet n-Butan, i-Butan und Propan sowie Mischungen aus diesen Kohlenwasserstoffen, ebenso geeignet ist beispielsweise aber auch Dimethylether. Bevorzugt wird eine Mischung aus n-Butan, i-Butan und Propan eingesetzt, wodurch die gewünschten, feinzelligen Schäume erhalten werden. Das Treibmittel oder das Treibmittelgemisch wird dabei typischerweise in einer Menge von 1 bis 50 Gew.-%, bevorzugt 5 bis 40 Gew.-% und besonders bevorzugt 5 bis 20 Gew.-% eingesetzt, wobei die Summe aus eingesetzter Polyurethan-Dispersion (I), anorganischem, kationischen Koagulans (II), Treibmittel (-gemisch) sowie gegebenenfalls eingesetzter Hilfs- und Zusatzstoffe (III) 100 Gew.-% ergibt. Die Bereitstellung der Sprühpflaster erfolgt bevorzugt in Sprühdose, wobei die Polyurethan-Dispersion (I) und das anorganische, kationische Koagulans (II) getrennt voneinander enthalten sind und unmittelbar vor der Applikation miteinander vermischt werden. In einer oder in beiden Komponenten kann das Treibmittel enthalten sein. Eine oder beide der Komponenten (I) bzw. (II) können darüber hinaus gegebenenfalls noch Hilfs- und Zusatzstoffe (III) enthalten, bevorzugt Schaumhilfsmittel. Neben dem Sprühen ist auch Gießen der Zusammensetzung möglich.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Verwendete Substanzen und Abkürzungen:

| | |
|---|---|
| Diaminosulfonat: | NH₂-CH₂CH₂-NH-CH₂CH₂-SO₃Na (45 %ig in Wasser) |
| | |
| Desmophen^{®} C2200: | Polycarbonatpolyol, OH-Zahl 56 mg KOH/g, zahlenmittleres Molekulargewicht 2000 g/mol (BayerMaterialScience AG, Leverkusen, DE) |
| | |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| | |
| PolyTHF^{®} 1000: | Polytetramethylenglykolpolyol, OH-Zahl 112 mg KOH/g, zahlenmittleres zahlenmittleres Molekulargewicht 1000 g/mol (BASF AG, Ludwigshafen, DE) |
| | |
| Polyether LB 25: | monofunktioneller Polyether auf Ethylenoxid-/Propylenoxidbasis, zahlenmittleres Molekulargewicht 2250 g/mol, OH-Zahl 25 mg KOH/g (BayerMaterialScience AG, Leverkusen, DE) |
| | |
| Impranil^{®} DLN: | aliphatische Polyesterpolyurethan-Dispersion, Feststoffgehalt 40 %, PH-Wert 6,6 (BayerMaterialScience AG, Leverkusen, DE) |

Die Bestimmung der mittleren Teilchengrößen (angegeben ist das Zahlenmittel) der Polyurethan-Dispersionen (I) erfolgte mittels Laserkorrelations-Spektroskopie (Gerät: Malvern Zetasizer 1000, Malver Inst. Limited).

### Beispiel 1: Polyurethan-Dispersion 1

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht oder leicht unterschritten war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 61 % |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

1077,2 g PolyTHF® 2000, 409,7 g PolyTHF® 1000, 830,9 g Desmophen® C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:

| | |
|---|---|
| Feststoffgehalt: | 61,6 % |
| Partikelgröße (LKS): | 528 nm |
| Viskosität (Viskosimeter, 23°C): | 166 mPas |
| pH (23°C): | 7,5 |

### Beispiele 3-11: Schäume, hergestellt aus den Polyurethan-Dispersionen der Beispiele 1-2 sowie Impranil^{®} DLN

Die in der Tabelle 1 angegebenen Mengen der kommerziell erhältlichen Polyurethan-Dispersion Impranil^{®} DLN bzw. der Polyurethan-Dispersionen, hergestellt wie in den Beispielen 1 und 2 beschrieben, wurden mit den Schaumhilfsmitteln sowie dem Verdicker wie dort ebenso angegeben vermischt und unter Verwendung eines handelsüblichen Handrührgerätes (Rührer aus gebogenem Draht) für 4 Minuten aufgeschlagen. Unter weiterem Rühren wurden die erhaltenen Schäume durch Zugabe der in Tabelle 1 angegebenen Salzlösungen schließlich koaguliert; durch die Koagulation blieb das Schaumvolumen nahezu unverändert (leichte Viskositätszunahme). Danach wurden die Schäume auf siliconbeschichtetes Papier ausgestrichen. Die anschließende Trocknung der Schäume erfolgte über Nacht bei Raumtemperatur. Es wurden durchweg reinweiße hydrophile Schäume mit guten mechanischen Eigenschaften und einer feinen Porenstruktur erhalten.

**Tabelle 1**

| Menge [g] | | | | | |
|---|---|---|---|---|---|
| Schaum Nr. | Polyurethan-Dispersion (Beispiel) | Schaumadditiv 1 | Schaumadditiv 2 | Koagulans (Konzentration in Wasser) | Gummi arabicum |
| 1 | 40,0 (1) | 0,5¹) | --- | 1,1 (70%)⁴⁾ | 0,8 |
| 2 | 40,0 (2) | 0,6¹⁾ | --- | 1,2 (70%)⁴⁾ | 0,8 |
| 3 | 40,0 (1) | 1,0²⁾ | 0,1³⁾ | 1,2 (70%)⁴⁾ | 0,9 |
| 4 | 40,0(2) | 1,1²⁾ | 0,1³⁾ | 1,3 (70%)⁵⁾ | 0,9 |
| 5 | 40,0 (1) | 0,5¹) | --- | 1,2 (70%)⁶⁾ | 0,8 |
| 6 | 40,0 (1) | 0,5¹) | --- | 1,2 (70%)⁶⁾ | 0,4 |
| 7 | 40,0 (Imprani^{®} DLN) | 0,5¹⁾ | --- | 2,3 (33%)⁵⁾ | 0,8 |
| 8 | 40,0 (Impranil^{®} DLN) | 0,5¹⁾ | --- | 2,9 (26%)⁷⁾ | 0,8 |
| 9 | 40,0 (Impranil^{®} DLN) | 0,5¹⁾ | --- | 2,6 (29%)⁸⁾ | 0,7 |

| | | | | | |
|---|---|---|---|---|---|
| 1) EO/PO-Blockcopolymer (Pluronic® PE 6800, BASF AG, Ludwigshafen, DE); 2) Alkylpolyglycosid (Plantacare^{®} 1200 UP, Cognis Deutschland GmbH & Co.KG, Düsseldorf, DE); 3) Ammoniumstearat (ca. 30 %, Stokal^{®} STA, Bozzetto GmbH, Krefeld, DE); 4) MgCl₂; 5) MgSO₄; 6) CaCl₂; 7) NaCl; 8) Al₂(SO₄)₃ | | | | | |

## Patentansprüche

1. Verfahren zur Herstellung von geschäumten Artikeln aus Polyurethan-Schäumen, bei dem eine Zusammensetzung enthaltend wässrige, anionisch hydrophilierte Polyurethan-Dispersionen (I) und anorganische kationische Koagulanzien (II) aufgeschäumt und getrocknet wird.

2. Verfahren gemäß Anspruch 1, **dadurch gekennzeichnet, dass** es sich bei dem geschäumten Artikel um eine Wundauflage handelt.

3. Verfahren gemäß Anspruch 1 oder 2, **dadurch gekennzeichnet, dass** die wässrigen, anionisch hydrophilierten Polyurethan-Dispersionen (I) erhältlich sind, indem
A) isocyanatfunktionelle Prepolymere aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und gegebenenfalls nichtionischen Hydrophilierungsmitteln,
hergestellt werden,
B) dessen freie NCO-Gruppen dann ganz oder teilweise
B1) gegebenenfalls mit aminofunktionellen Verbindungen mit Molekulargewichten von 32 bis 400 g/mol und
B2) aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

4. Verfahren gemäß Anspruch 3, **dadurch gekennzeichnet, dass** bei der Herstellung der wässrigen, anionisch hydrophilierten Polyurethan-Dispersionen (I) in A1) 1,6-Hexamethylendiisocyanat, Isophorondiisocyanat, die isomeren Bis-(4,4'-isocyanatocyclohexyl)methane, sowie deren Mischungen eingesetzt werden und in A2) eine Mischung aus Polycarbonatpolyolen und Polytetramethylenglykolpolyolen eingesetzt wird, wobei der Anteil der Summe der Polycarbonat- und Polytetramethylenglykolpolyetherpolyole an der Komponente A2) mindestens 70 Gew.-% beträgt.

5. Verfahren gemäß einem der Ansprüche 1 bis 4, **dadurch gekennzeichnet, dass** das anorganische kationische Koagulans (II) ein wasserlösliches anorganisches Salz ist.

6. Verfahren gemäß Anspruch 5, **dadurch gekennzeichnet, dass** das wasserlösliche anorganische Salz ein Erdalkalimetallsalz ist.

7. Verfahren gemäß Anspruch 5 oder 6, **dadurch gekennzeichnet, dass** das wasserlösliche anorganische Salz Magnesiumchlorid, Calciumchlorid oder eine Mischung aus beiden Salzen ist.

8. Verfahren gemäß einem der Ansprüche 1 bis 7, **dadurch gekennzeichnet, dass** neben der Polyurethan-Dispersion (I) und dem anorganischen kationischen Koagulans (II) auch Hilfs- und Zusatzstoffe (III) enthalten sind.

9. Verfahren gemäß Anspruch 8, **dadurch gekennzeichnet, dass** als Hilfs- und Zusatzstoffe (III) Fettsäureamide, Sulfosuccinamide, Kohlenwasserstoffsulfonate, -sulfate, Fettsäuresalze, EO/PO-Blockcopolymere und/oder Alkylpolyglycoside als Schaumbildner und - stabilisatoren enthalten sind.

10. Verfahren gemäß Anspruch 9, **dadurch gekennzeichnet, dass** als Schaumbildner und -stabilisatoren ausschließlich EO/PO-Blockcopolymere enthalten sind.

11. Verfahren gemäß einem der Ansprüche 1 bis 10, **dadurch gekennzeichnet, dass** Wirkstoffe aus der Gruppe der Antiseptika, Wachstumsfaktoren, Proteaseinhibitoren und nichtsteroidalen Antiphlogistika/Opiate mit verwendet werden.

12. Verfahren gemäß Anspruch 11, **dadurch gekennzeichnet, dass** als Wirkstoff ein antiseptisches Biguanid und/oder dessen Salz verwendet wird.

13. Geschäumte Artikel erhältlich nach einem Verfahren gemäß einem der Ansprüche 1 bis 12.

14. Geschäumte Artikel gemäß Anspruch 13, **dadurch gekennzeichnet, dass** diese eine mikroporöse, offenporige Struktur aufweisen und eine Dichte in getrocknetem Zustand von unterhalb 0,4 g/cm³ aufweisen.

15. Geschäumte Artikel gemäß Anspruch 13 oder 14, **dadurch gekennzeichnet, dass** diese ein Absorptionsvermögen gegenüber physiologischer Salzlösung von 100 bis 1500 % (Masse der aufgenommenen Flüssigkeit bezogen auf die Masse des trockenen Schaums; Bestimmung nach DIN EN 13726-1, Teil 3.2) und eine Durchlässigkeit gegenüber Wasserdampf von 500 bis 8000 g/24 h * m² besitzen (Bestimmung nach DIN EN 13726-2, Teil 3.2).

16. Geschäumte Artikel gemäß einem der Ansprüche 13 bis 15, **dadurch gekennzeichnet, dass** es sich um eine Wundauflage handelt.

17. Zusammensetzungen enthaltend eine wässrige, anionisch hydrophilierte Polyurethan-Dispersion (I) und ein anorganisches kationisches Koagulans (II).

18. Zusammensetzungen gemäß Anspruch 17, **dadurch gekennzeichnet, dass** diese ferner Wirkstoffe aus der Gruppe der Antiseptika, Wachstumsfaktoren, Proteaseinhibitoren und nichtsteroidalen Antiphlogistika/Opiate umfassen.

19. Zusammensetzungen gemäß Anspruch 18, **dadurch gekennzeichnet, dass** als Wirkstoff ein antiseptisches Biguanid und/oder dessen Salz enthalten ist.
